# EUROPEAN PATENT APPLICATION

(11) **EP 1 780 543 A1**
(43) Date of publication of application: **02.05.2007**
(21) Application number: 05765777.7
(22) Date of filing: 13.07.2005
(51) Int. Cl.: G01N 33/53, G01N 33/531, G01N 33/543

(54) **METHOD OF REMOVING ADHESIVE MICROVESICLES**

(30) Priority: 26.07.2004 JP 2004217387
(71) Applicant: Otsuka Pharmaceutical Co., Ltd., Tokyo 101-8535 (JP); Jimro Co., Ltd., Takasaki-shi, Gunma 370-0021 (JP)
(72) Inventor: OZEKI, Yasushi, Chofu-shi, Tokyo 1820023 (JP); SAITOU, Fumio, Takasaki-shi, Gunma 3701213 (JP)
(74) Representative: HOFFMANN EITLE
(86) International application number: PCT/JP2005/012932
(87) International publication number: WO 2006/011363

(57) **Abstract**

A method for selectively measuring microvesicles alone in blood by pretreating a sample derived from blood with albumin to selectively eliminate microvesicles alone released from platelets activated by an artificial manipulation.

## Description

### TECHNICAL FIELD

The present invention relates to a method for pretreating adhesive microvesicles produced in a sample derived from blood for measuring microvesicles not to affect measurement of circulating microvesicles present in the blood before blood sampling, and a method for correctly measuring the circulating microvesicles, and a reagent or a kit for the measurement thereof.

### BACKGROUND ART

Microvesicles are released from platelets activated when thrombus is formed in atherosclerosis, disseminated intravascular coagulation syndrome and the like. For the microvesicles, various functions such as a blood coagulation accelerating effect by binding/condensing coagulation factors to phospholipid on membrane surfaces, an activation of monocytes, platelets and vascular endothelial cells and an adherence accelerating effect of leukocytes-platelets/leukocytes-leukocytes/endothelial cells-leukocytes have been known. Thus, the microvesicle is believed to be not only a marker for the platelet activation but also be deeply involved in thrombus formation and progress of arteriosclerosis (see Non-Patent Document 1). Therefore, it is one of important diagnostic procedures for presuming with time a condition of a disease associated with the above events to measure an amount of microvesicles in the blood. However, the platelets are easily activated by physical stimulation, and based on this, the microvesicles are released from the activated platelets. For example, because of physical shock at blood sampling or during separation of plasma or serum, contaminated platelets are activated thereby increasing the microvesicles which do not reflect a clinical situation in a sample derived from the blood after the blood sampling. Such microvesicles boost the amount of the microvesicles present in the sample derived from the blood, and cause a trouble for accurately determining the condition of a patient. Therefore, a method for accurately measuring the microvesicles in the blood before the blood sampling by eliminating an influence of the microvesicles produced after the blood sampling has been required.

Non-patent Document 1: Nomura S., Int. J. Hematol., 74:397-404, 2001

### DISCLOSURE OF INVENTION

### PROBLEMS TO BE SOLVED BY THE INVENTION

In order to solve the above problem caused by the microvesicles (adhesive microvesicles) produced upon the preparation of a sample derived from the blood after blood sampling, it is an object of the present invention to provide a method for removing the adhesive microvesicles, a method for correctly measuring circulating microvesicles in blood by taking advantage of this, and a reagent for the measurement thereof.

### MEANS FOR SOLVING THE PROBLEMS

In the light of such an actual circumstance, as a result of an extensive study, the present inventors have found that albumin typified by BSA (bovine serum albumin) specifically interacts with microvesicles (adhesive microvesicles) produced after blood sampling to eliminate their influence on immunological assays, and helps measure correctly microvesicles (circulating microvesicles) alone present before the blood sampling by the immunological assay. The present invention has been completed based on the aforementioned results, and specifically provides the following inventions.
[1] A method for pretreating a sample derived from blood for eliminating an influence of adhesive microvesicles produced after blood sampling in the sample derived from the blood, characterized in that albumin is added into the sample derived from the blood.
[2] The method according to [1] wherein the albumin is BSA.
[3] An immunoassay method of circulating microvesicles in a sample derived from blood comprising a step of eliminating an influence of adhesive microvesicles produced after blood sampling in the sample derived from the blood by adding albumin into the sample derived from the blood and a step of immunologically assaying the circulating microvesicles in the sample derived from the blood.
[4] The method according to [3] wherein the circulating microvesicles are immunologically assayed by coupling an antibody against the circulating microvesicle to a surface epitope specific for the microvesicle and measuring a level of the consequently bound antibody.
[5] The method according to [4] wherein the above surface epitope is present on glycoprotein of the circulating microvesicle.
[6] The method according to [5] wherein the above surface glycoprotein is GpIb-IX.
[7] The method according to [6] characterized in that the antibody is detected based on a label or an enzyme conjugated to an antibody.
[8] The method according to [7] characterized by comprising a procedure wherein the circulating microvesicles are bound to an immobilized antibody against GpIX and subsequently an antibody which recognizes the GpIb on the microvesicles is reacted.
[9] A kit for measuring circulating microvesicles in a sample derived from blood comprising at least albumin, a labeled anti-GpIb antibody and an anti-GpIX antibody.
[10] The kit according to [9] wherein the albumin is BSA.
[11] The kit according to [9] or [10] wherein the anti-GpIX antibody is coated in a plate.

Herein, the microvesicle present in the blood of the patient is referred to as the "circulating microvesicle", and the microvesicle released from the platelet activated by an artificial manipulation during or after the blood sampling is referred to as the "adhesive microvesicle" because of its affinity to albumin.

The sample derived from the blood subjected in the present invention indicates a solution comprising the blood or a blood component(s) prepared from the blood, and is preferably the sample from which the platelets have been removed. Specifically, the sample can include plasma, and also comprises solutions obtained by diluting with saline or buffer in terms of measurement of the blood fraction.

The albumin indicates albumins derived from human, mammalian animals other than human and birds, and the mammalian animals other than the human can include, for example, cattles, horses, monkeys, dogs, rabbits and mice, and the birds can include chicken as representative examples. In particular, bovine serum albumin (BSA) can be include as the representative example, and additionally, human serum albumin (HSA) and ovalbumin can be included as suitable examples.

For a timing to add the albumin, the albumin may be added to the sample derived from the blood before the measurement, and for example in an ELISA method, the albumin may be added after immobilizing an antibody onto a plate. An addition form is not particularly limited, but it is desirable to add in a liquid state, and the albumin has been previously dissolved in the saline or an isotonic buffer and then can be added. An amount of the albumin to be added is desirably 5% or more at a final concentration, and for example, the final concentration of about 5 to 10% is exemplified. It is desirable to leave stand or shake for allowing the adhesive microvesicles to absorb to the albumin after the addition, and it is desirable that the treatment with albumin is performed for one hour or more and suitably 4 hours or more. Thus, the microvesicles released from the artificially activated platelets interact with the albumin, are eliminated out of the system for the immunoassay by the antibody which takes advantage of a microvesicle membrane surface antigen, and have no substantial influence on the measurement of the circulating microvesicles.

The treatment with albumin does not substantially affect the original circulating microvesicles in the sample derived from the blood, and the circulating microvesicles can be measured by the standard immunoassay which takes advantage of the microvesicle property.

The immunoassay is not particularly limited as long as it is the procedure in which the microvesicles are measured and quantified based on an antigenic molecule (epitope) on the membrane surface of the microvesicle by taking advantage of an antibody against the epitope and quantifying the molecules. For example, an ELISA method, a RIA method or an aggregation method can be exemplified.

Preferable antibodies according to the present invention can include antibodies against a GpIb-GPIX complex and/or glycoproteins including individual glycoprotein, GpIb or GpIX. Other glycoproteins specific for the platelets observed on the microvesicles include GpIa-IIa, GpIIb-IIIa and GpIIIb.

The antibody itself bound to the circulating microvesicle may be labeled, or a labeled second antibody, e.g., a labeled anti-mouse immunoglobulin specifically bound to a mouse monoclonal antibody (first antibody) may be used. Either a polyclonal antibody or a monoclonal antibody may be used, but preferably the monoclonal antibody can be used. The labels may be any component which can give a signal, e.g., enzymes (peroxidase, alkali phosphatase, etc.), chromophores, fluorophores (FITC, etc.), radioisotopes, colored particles, dyes, colloidal metals and the like.

Furthermore, one embodiment of the present invention will be described in detail below by taking the ELISA method using BAS for instance.

An aliquot of 10 to 150 µL of the component derived from the blood prepared as the above is added into each well of a 96-well plate to which an anti-GpIX antibody has been previously coated and blocking with skim milk has been given, and then, a BSA solution is added thereto at a final concentration of 5%. Thereafter, in order to adsorb newly produced adhesive microvesicles to BSA, the plate is incubated at room temperature or at 37°C for one hour or more, preferably 4 hours or more. Subsequently, the plate is thoroughly washed with a washing solution, e.g., about 0.02 to 0.1% Tween 20/PBS. Then, a peroxidase-labeled anti-GpIb antibody is added. The plate is thoroughly washed with the same washing solution. Subsequently a substrate for the enzyme is added to develop a color. Thus, it becomes possible to quantify the amount of the circulating microvesicles as the amount of colored substrate developed by an enzymatic reaction, by taking advantage of the antibodies based on the membrane surface epitope GpIb-GpIX complex. The adhesive microvesicles produced by the artificial manipulation are trapped by BSA through the above manipulation and become non-reactive with the antibodies used in a series of immunoassay. Thus, it is possible to measure only the objective circulating microvesicles originally present in the blood.

A kit for measuring the circulating microvesicles comprising various antibodies and reagents and the like used for the above manipulation can be provided. Specifically, albumin typified by BSA or the solution thereof for efficiently removing the adhesive microvesicles produced by the artificial manipulation, the antibodies which recognize the protein complex, typified by the antibodies against GpIb and GpIX on the membrane surface of the microvesicle (either or both of the antibodies are labeled), if necessary the second antibody and the enzyme substrate (when the enzyme is used as the label) and the like are included in the kit. For example, when the antibody for recognition is a biotin-conjugated anti-GpIb antibody, peroxidase-labeled avidin can be included as the second reagent in the kit for measurement. The kit for measuring the circulating microvesicles comprising the substrate for the peroxidase can be provided.

As is evident from the following Example, it has been found that the adhesive microvesicles conventionally released from the activated platelets contaminated at a stage of blood processing can be efficiently removed from the immunoassay system by pretreating with albumin and that only the circulating microvesicles originally present in the blood can be measured.

### BRIEF DESCRIPTION OF DRAWINGS

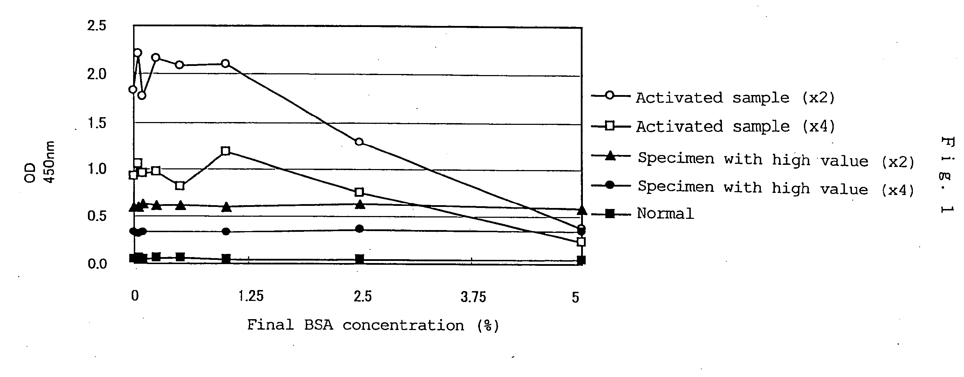

Fig. 1 is a view showing an absorption test of adhesive microvesicles by BSA using artificially activated samples, samples derived from a patient with high microvesicles and a normal sample.

### BEST MODE FOR CARRYING OUT THE INVENTION

The present invention will be described in more detail with reference to an Example. This Example is described for a mere illustration and does not limit the invention.

### Example 1

### Methods:

### 1. Preparation of pseudopositive (activated) samples

Whole blood sampled with 3.8% citric acid (citrate) from a healthy adult male who showed a normal value of circulating microvesicles (PDMP: platelet-derived microparticles) was stored in a refrigerator at 4°C for 12 hours to newly produce PDMP (adhesive microvesicles). The cooled whole blood (2 mL) was centrifuged at 3000 rpm for 20 min by a desktop centrifuge and a supernatant (600 µL) was collected to make an activated sample. The activated sample was diluted with saline to 2 times and 4 times to use for ELISA.

### 2. Preparation of specimen with high value

The blood (2 mL) was sampled with EDTA/ACD (acid citrate dextrose) from a subject (adult male) who showed a high PDMP value, centrifuged at 3000 rpm at room temperature for 20 min by a desktop centrifuge and a supernatant (600 µL) was collected. The specimen with high value was diluted with saline to 2 times and 4 times to use for ELISA.

### 3. Preparation of normal specimen

The blood was sampled with EDTA/ACD from a healthy adult male who showed a normal PDMP value, centrifuged at 3000 rpm for 20 min by a desktop centrifuge and a supernatant (600 µL) was collected to use for ELISA.

### 4. ELISA

A sandwich ELISA system was made using antibodies (anti-CD42b antibody: NNKY5-5, anti-CD42a antibody: KMP-9) against CD42b (GpIb) and CD42a (GpIX) which were molecules specific for platelets in glycoproteins present on membrane surface of the microvesicle. An aliquot of 50 µL of the activated sample or the specimen with high value was added to each well of a 96-well microtiter plate for ELISA (supplied from Corning: MaxiSorb) to which the anti-CD42a antibody had been coated, and subsequently 50 µL of PBS, 2.5%, 5% , 7.5% or 10% BSA was added to each well to make a final concentration of BSA 1.25%, 2.5%, 3.75% or 5%. The plate was incubated at room temperature for 4 hours with shaking. Then, the plate was washed with a washing solution (0.05% Tween 20/PBS), the biotinylated anti-CD42b antibody was added, the color was developed by adding peroxidase-labeled avidin, and absorbance at 450 nm was read out by an immunoreader (Fig. 1)

### Results

In the activated sample which exhibited the pseudopositive, the measured values were decreased depending on the concentrations of BSA added in the wells in the assay, and the value at a final BSA concentration of 5% was lowered to close to the value of the normal specimen. Meanwhile, the specimen with high value which had primarily exhibited the high value exhibited constant values regardless of the BSA concentrations. From these results, it has been confirmed that the PDMP newly produced by the manipulation after the blood sampling can be selectively removed by adding BSA at a final concentration of 5% in the assay.

## Claims

1. A method for pretreating a sample derived from blood for eliminating an influence of adhesive microvesicles produced after blood sampling in the sample derived from the blood,
**characterized in that** albumin is added into the sample derived from the blood.

2. The method according to claim 1 wherein the albumin is BSA.

3. An immunoassay method of circulating microvesicles in a sample derived from blood comprising a step of eliminating an influence of adhesive microvesicles produced after blood sampling in the sample derived from the blood by adding albumin into the sample derived from the blood and a step of immunologically assaying the circulating microvesicles in the sample derived from the blood.

4. The method according to claim 3 wherein the circulating microvesicles are immunologically assayed by coupling an antibody against the circulating microvesicle to a surface epitope specific for the microvesicle and measuring a level of the consequently bound antibody.

5. The method according to claim 4, wherein said surface epitope is present on glycoprotein of the circulating microvesicle.

6. The method according to claim 5 wherein said surface glycoprotein is GpIb-IX.

7. The method according to claim 6 wherein the antibody is detected based on a label or an enzyme conjugated to the antibody.

8. The method according to claim 7 **characterized by** comprising a procedure wherein circulating microvesicles are bound to an immobilized antibody against GpIX and subsequently an antibody which recognizes the GpIb on the microvesicles is reacted.

9. A kit for measuring circulating microvesicles in a sample derived from blood comprising at least albumin, a labeled anti-GpIb antibody and an anti-GpIX antibody.

10. The kit according to claim 9 wherein the albumin is BSA.

11. The kit according to claim 9 or 10 wherein the anti-GpIX antibody is coated in a plate.
